# EUROPÄISCHE PATENTSCHRIFT

(11) **EP 2 384 708 B1**
(45) Veröffentlichungstag und Bekanntmachung des Hinweises auf die Patenterteilung: **18.12.2013**
(21) Anmeldenummer: 11165017.2
(22) Anmeldetag: 05.05.2011
(51) Int. Cl.: A61B 17/24, A61M 25/01, A61M 25/06, A61M 29/02, A61M 25/00, A61M 29/00

(54) **Einführvorrichtung für einen Ballonkatheter**
Insertion device for a balloon catheter
Dispositif d'introduction pour un cathéter à ballonnet

(30) Priorität: 06.05.2010 DE 202010005388 U
(43) Veröffentlichungstag der Anmeldung: 09.11.2011
(73) Patentinhaber: Spiggle & Theis Medizintechnik GmbH, 51491 Overath (DE)
(72) Erfinder: Spiggle, David, 53819 Neunkirchen-Seelscheid (DE); Theis, Detlef, 51674 Wiehl (DE)
(74) Vertreter: Fuchs

(56) Entgegenhaltungen:
- WO-A1-2008/156705
- WO-A1-2010/033629
- US-A1- 2004 082 905
- US-A1- 2005 149 159
- US-A1- 2007 156 225

## Beschreibung

Die vorliegende Erfindung betrifft eine Einführvorrichtung für einen Ballonkatheter, umfassend einen rohrförmigen Grundkörper und einen in den rohrförmigen Grundkörper einbringbaren und im rohrförmigen Grundkörper verschiebbaren Schaft zum Bewegen des Ballonkatheters. Derartige Ballonkatheter werden immer dann eingesetzt, wenn sich Gefäße oder Röhren im menschlichen oder tierischen Körper verengt haben und geweitet werden müssen. Eine derartige Röhre ist beispielsweise die Eustachische Röhre. Die Eustachische Röhre *(Tuba auditiva Eustachii)* verbindet die Paukenhöhle des Ohres mit dem Nasenrachen und ermöglicht einen Druckausgleich, so dass der Druck im Mittelohr dem des Nasen-Rachen-Raums und somit dem Außendruck angeglichen wird. Ist die Eustachische Röhre verengt oder gar geschlossen, so kann der Druckausgleich nicht oder nicht mehr in ausreichendem Maße stattfinden, was zu erheblichen Schmerzen, Verschlechterung des Hörvermögens und zu Entzündungen und Schädigungen des Mittelohres führen kann.

Ein Ballonkatheter weist an seinem distalen Ende einen Ballon auf, welcher bei Bedarf durch Einfüllen von steriler Kochsalzlösung expandiert werden kann. Um die Verengung der Eustachischen Röhre zu beseitigen, wird der Ballonkatheter mit dem Ende, an dem sich der Ballon befindet, minimal-invasiv/endoskopisch über den Nasenrachen in die Eustachische Röhre eingeführt. Der Ballon ist solange nicht expandiert, bis er die gewünschte Stelle erreicht hat. Dann wird der Ballon unter Druck gesetzt, so dass sein Volumen zunimmt, wodurch die verengte Stelle der Eustachischen Röhre erweitert wird. Der Ballon wird für ca. zwei Minuten bei einem Druck von etwa 10 bar im aufgeblasenen Zustand gehalten, wodurch die Verengung beseitigt werden kann. Anschließend wird der Druck abgelassen, so dass sich das Volumen des Ballons verringert. Dann wird der Ballonkatheter aus der Eustachischen Röhre und dem Nasenrachen entfernt, womit die Behandlung abgeschlossen ist. Diese Art und Weise der Behandlung ist minimal-invasiv und wenig traumatisch, weshalb sie das Mittel der Wahl zur Beseitigung der Verengung der Eustachischen Röhre bzw. von Tuben-Belüftungsstörungen darstellt.

Für einen erfolgreichen Verlauf der Behandlung ist es aber von höchster Wichtigkeit, dass der Ballonkatheter und damit der Ballon an die richtige Stelle im Körper, beispielsweise in der Eustachischen Röhre, gebracht werden. Wird der Ballonkatheter nicht weit genug in die Eustachische Röhre eingebracht, so besteht die Gefahr, dass die Behandlung ohne Effekt bleibt, da an der Stelle, wo der Ballon unter Druck gesetzt worden ist, keine Verengung vorliegt. Wird er hingegen zu weit eingebracht, können irreparable Verletzungen von angrenzenden Körperteilen erzeugt werden.

Aus der DE 20 2006 017 173 U1 ist ein Uretero-Renoskop bekannt, das ein starres Rohr umfasst, in welchem ein Katheter eines Endoskops verschiebbar angeordnet ist. Das starre Rohr ist über einen Schieber an einem Verbindungsstück befestigt. Der Schieber kann gegenüber dem Verbindungsstück verschoben werden, allerdings bewirkt ein Verschieben des Schiebers keine Verschiebung des Katheters im starren Rohr. Somit kann die Position des Katheters im starren Rohr nicht bestimmt werden, wodurch es auch nicht möglich ist, die Position des Endes des Katheters, welches in die Röhren eingeführt wird, genau zu bestimmen.

WO 2010/033629A1, US2004/0082905A1, US2005/0149159A1 und US2007/0156225A1 offenbaren ähnliche Geräte.

Davon ausgehend liegt der Erfindung die Aufgabe zugrunde, die gattungsgemäße Einführvorrichtung für Ballonkatheter so weiterzuentwickeln, dass die Position des Endes des Katheters, welches in die Gefäße oder Röhren des menschlichen oder tierischen Körpers eingeführt wird und an dem der Ballon angeordnet ist, bestimmbar ist und insbesondere verhindert wird, dass der Katheter über eine maximale Eindringtiefe hinaus in die Röhren eingeführt wird. Gelöst wird die Aufgabe mit einer Einführvorrichtung nach Anspruch 1. Der Ballonkatheter wird mit dem Schaft bewegt, so dass die Position des Schafts im rohrförmigen Grundkörper einen direkten Rückschluss auf die Position des Ballonkatheters in den Röhren zulässt, in welche er eingeführt worden ist. Mittels der Einrichtung kann jederzeit genau festgestellt werden, wie weit der Ballonkatheter in die Röhre eingeschoben worden ist. Weiterhin kann festgestellt werden, ob sich der Ballon an der gewünschten Position befindet, so dass die Behandlung mit einem Maximum an Effektivität durchgeführt werden kann, während gleichzeitig verhindert wird, dass angrenzende Organe oder Körperteile verletzt werden.

Vorzugsweise umfasst die Einrichtung eine die Wandung des rohrförmigen Grundkörpers vollständig durchdringende Ausnehmung zum Ermitteln der Position des Schafts. Die Ausnehmung ermöglicht auf konstruktiv einfache Weise, die Position des Schafts im rohrförmigen Grundkörper zu bestimmen, da sie den Blick durch den rohrförmigen Grundkörper direkt auf den Schaft freigibt. Der behandelnde Operateur kann jederzeit einen Rückschluss auf die Position des Ballonkatheters im Gefäß oder in der Röhre ziehen, ohne dass aufwendige Geräte zur Ermittlung der Position des Schafts im rohrförmigen Grundkörper vorgesehen werden müssen.

In einer bevorzugten Ausgestaltung der vorliegenden Einführvorrichtung umfasst der Schaft einen mit der Ausnehmung zusammenwirkenden Anschlag zum Definieren einer ersten Endstellung des Schafts im rohrförmigen Grundkörper. Mit der Definition einer ersten Endstellung wird eine maximale Länge festgesetzt, über die hinaus der Ballonkatheter nicht in die Röhre eingeführt werden kann. Dies ist insbesondere dann vorteilhaft, wenn sich in unmittelbarer Nachbarschaft zu dem zu behandelnden Gefäß oder der Röhre empfindliche Körperteile befinden, deren Verletzung bei einer zu weiten Einführung des Ballonkatheters irreparable Folgen haben würde. Üblicherweise wird hierzu eine Untersuchung per Computer-Tomographie (CT) durchgeführt. Diese Ausgestaltung leistet somit einen Beitrag zur Behandlungssicherheit.

Bevorzugt ist der Anschlag als eine Feder ausgeführt, die in eine korrespondierende Nut des Schafts einbringbar ist. Dieser Aufbau ist konstruktiv besonders einfach, weiterhin wird die Montage und Demontage der Einführvorrichtung erleichtert, was bei medizinischen Geräten hinsichtlich der Reinigung und Sterilisation von hoher Wichtigkeit ist.

Die Ausnehmung weist ein offenes Ende und ein geschlossenes Ende auf, wobei der Schaft mit dem Anschlag durch das offene Ende in den rohrförmigen Grundkörper einbringbar ist. Hierdurch werden die Montage und die Demontage der Einführvorrichtung vereinfacht, so dass die Einführvorrichtung schnell zum Autoklavieren zerlegt und vor oder während der Behandlung zusammengesetzt werden kann.

Eine bevorzugte Ausgestaltung umfasst ein Verschlusselement, mit dem das offene Ende wahlweise verschließbar ist. Das Verschlusselement kann einfach und schnell geöffnet und geschlossen werden, so dass die Montage und Demontage weiter vereinfacht wird. Weiterhin verhindert das Verschlusselement, dass der Schaft versehentlich aus dem rohrförmigen Grundkörper entnommen wird.

Vorteilhafterweise ist das Verschlusselement als eine Überwurfmutter ausgebildet, die auf ein Gewinde auf einer Außenfläche des rohrförmigen Grundkörpers aufschraubbar ist. Diese Ausgestaltung des Verschlusselements ist aus fertigungstechnischer Sicht besonders einfach und kostengünstig, weiterhin ist die Überwurfmutter einfach zu bedienen und zeichnet sich durch eine hohe Zuverlässigkeit aus.

Weiterhin ist es bevorzugt, dass die Einrichtung eine Skalierung zum Ermitteln der Position des Schafts im rohrförmigen Grundkörper aufweist. Die Skalierung ist vorzugsweise auf der Außenfläche des rohrförmigen Grundkörpers angeordnet und gibt dem Operateur eine Hilfestellung, um eine quantitative und reproduzierbare Angabe bezüglich der Position des Schafts im rohrförmigen Grundkörper und damit des Ballonkatheters im Gefäß oder in der Röhre zu machen. Weiterhin können Vorschubprofile erstellt und einfach umgesetzt werden. Ist es beispielsweise bekannt, dass in einem vor der zu behandelnden Verengung des Gefäßes oder der Röhre liegenden Abschnitt keine empfindlichen Körperteile angeordnet sind, kann in diesem Abschnitt der Ballonkatheter schnell vorgeschoben werden. Bei Annäherung an die zu behandelnde Verengung kann der Schaft langsamer vorgeschoben werden, womit der Ballonkatheter behutsamer im Gefäß oder der Röhre bewegt wird, wodurch die Wahrscheinlichkeit, ein benachbartes Körperteil zu verletzen, verringert wird.

Weiterhin ist es von Vorteil, wenn die Feder eine mit der Skalierung zusammenwirkende Markierung zum Ermitteln der Position des Schaftes im rohrförmigen Grundkörper aufweist. Die Markierung hebt sich visuell vom übrigen Schaft deutlich ab, so dass die Position des Schafts im rohrförmigen Körper vom Operateur einfach und eindeutig ermittelt werden kann, was einen Beitrag zur Sicherheit der Behandlung leistet.

Vorteilhafterweise definieren das geschlossene Ende der Ausnehmung die erste Endstellung und die Überwurfmutter eine zweite Endstellung, innerhalb derer der Schaft im rohrförmigen Grundkörper verschiebbar ist. Hierbei lässt sich auf konstruktiv einfache Weise eine Start- und eine Endposition definieren, zwischen denen der Ballonkatheter hin- und her verschiebbar ist, wobei die zweite Endstellung die Startposition vorgibt, von der aus der Ballonkatheter in das zu behandelnde Gefäß oder die Röhre eingeschoben wird. Der Ballonkatheter kann über den Abstand zwischen der ersten und zweiten Endstellung hinaus nicht in das Gefäß oder in die Röhre eingebracht werden. Der Operateur hat die Sicherheit, dass er den Ballonkatheter nicht ungewollt weiter in das Gefäß oder die Röhre einführen kann als vorgesehen. Somit wird ein Beitrag zur Sicherheit der Behandlung geleistet.

In einer bevorzugten Ausgestaltung der vorliegenden Erfindung beträgt der Abstand zwischen der ersten und der zweiten Endstellung zwischen 0 und 40 mm, insbesondere zwischen 0 und 35 mm. Die Länge der Eustachischen Röhre beträgt üblicherweise zwischen 31 und 38 mm, so dass verhindert wird, dass beim Einführen des Ballonkatheters in die Eustachische Röhre angrenzende Körperteile verletzt werden, beispielsweise das Trommelfell oder die Ossikel.

Eine vorteilhafte Ausgestaltung der erfindungsgemäßen Einführvorrichtung ist durch eine Einstelleinrichtung gekennzeichnet, mit welcher die maximale Verschiebbarkeit des Schafts im rohrförmigen Grundkörper einstellbar ist. Somit lässt sich die Einführvorrichtung für verschiedene Bereiche des Körpers verwenden, die mit einem Ballonkatheter behandelt werden sollen. Weiterhin können individuelle anatomische Eigenschaften der zu behandelnden Person genau berücksichtigt werden. Wie oben dargelegt, beträgt die Länge der Eustachischen Röhre zwischen 31 und 35 mm, was aber nur für Erwachsene gilt und wobei Abweichungen bei Anomalien möglich sind. Die Länge der Eustachischen Röhre kann beispielsweise mittels einer Computer-Tomographie ermittelt und bei der Wahl der maximalen Verschiebbarkeit des Schafts im rohrförmigen Grundkörper berücksichtigt werden.

Vorzugsweise umfasst die Einstellvorrichtung eine Mutter, die auf ein weiteres Gewinde auf einer Außenseite des Schafts aufschraubbar ist. Die erste Endstellung wird durch Anschlagen der Mutter an den rohrförmigen Grundkörper definiert. Durch Drehen der Mutter wird deren Position auf dem Gewinde verändert, wodurch auch die erste Endstellung verändert werden kann. Diese Ausgestaltung der erfindungsgemäßen Einstellvorrichtung lässt sich fertigungstechnisch auf einfache Weise herstellen, weiterhin ist sie robust und lässt sich zum Autoklavieren einfach zerlegen und anschließend wieder zusammensetzen.

Ferner umfasst eine Ausgestaltung der erfindungsgemäßen Einführvorrichtung eine Fixiereinrichtung zum Fixieren des Schaftes im rohrförmigen Grundkörper. Ist die erste Endstellung erreicht und der Ballonkatheter in die gewünschte Stelle im zu behandelnden Gefäß oder in der Röhre gebracht worden, wird mittels der Fixiereinrichtung die Position des Schaftes im rohrförmigen Grundkörper fixiert, so dass sie sich während der eigentlichen Behandlung, also beim Dilatieren des Ballons mittels der sterilen Kochsalzlösung, nicht verändert. Es wird ein Beitrag zur Sicherheit der Behandlung geleistet.

In einer bevorzugten Weiterentwicklung weist die vorliegende erfindungsgemäße Einführvorrichtung eine Greifeinrichtung zum Verschieben des Schaftes im rohrförmigen Grundkörper auf. Die Greifeinrichtung erleichtert dem Operateur die Verschiebung des Schafts im rohrförmigen Grundkörper, wodurch er eine bessere Kontrolle über den Behandlungsvorgang hat.

Vorteilhafterweise weist die Greifeinrichtung eine Anzahl von Ringen auf, von der mindestens ein Ring am rohrförmigen Grundkörper und mindestens ein Ring am Schaft befestigt sind. Der Operateur kann seine Finger in die Ringe einbringen und somit den Schaft bequem in Richtung der beiden Endstellungen hin-und her verschieben, so dass er sowohl den Einführ- als auch den Entfern-Vorgang ohne Umfassen und Abrutschen mit einer Hand durchführen kann.

Eine weitere Ausführungsform der erfindungsgemäßen Einführvorrichtung ist gekennzeichnet durch eine Anschlusseinrichtung zum lösbaren Anschließen einer weiteren Einheit an die Einführvorrichtung. Die Anschlusseinheit kann eine Zusammenführeinrichtung sein, mit der beispielsweise ein Lichtkanal und ein Beobachtungskanal zusammen mit dem Ballonkatheter in das zu behandelnde Gefäß oder in die zu behandelnde Röhre eingeführt werden können. Diese Ausführungsform ermöglicht es dem Operateur, den Vorschub des Ballonkatheters und die Behandlung der Verengung des Gefäßes oder der Röhre zu beobachten und zu kontrollieren. Der Lichtkanal und der Beobachtungskanal sind typische Bestandteile eines Endoskops, welches für minimal-invasive Eingriffe regelmäßig eingesetzt wird. Hierdurch wird ein höchstes Maß an Kontrolle der Behandlung ermöglicht. Die weitere Einheit kann aber auch so ausgeführt sein, dass keine weiteren Geräte wie ein Endoskop angeschlossen werden können. In diesem Fall können der Vorschub des Ballonkatheters und die Behandlung von einer weiteren, externen Beobachtungseinheit verfolgt werden.

Vorzugsweise besteht die Einführvorrichtung aus sterilisierbarem Material. Bei medizinischen Geräten, welche bei Operationen eingesetzt werden, ist die Vermeidung der Übertragung von Krankheitserregern sehr wichtig, weshalb diese so keimfrei wie möglich sein müssen. Eine Sterilisierung wird üblicherweise mithilfe eines Autoklaven vorgenommen, in dem die medizinischen Geräte unter einem bestimmten Druck in einer Wasserdampfatmosphäre für eine bestimmte Zeit behandelt werden. Diese Behandlung stellt eine hohe Belastung an das zu sterilisierende Material dar, weshalb nicht jedes Material verwendet werden kann. Durch die Wahl des geeigneten Materials kann die Sterilisierung wiederholt durchgeführt werden, womit die erfindungsgemäße Einführvorrichtung kein Einweg-Produkt darstellt und einen Beitrag zur Nachhaltigkeit liefert.

Ferner kann auch eine chemische Sterilisation (auch Gassterilisation genannt) durchgeführt werden, die mit chemischen Stoffen, wie z.B. Formaldehyd, Ethylenoxid oder Peressigsäure durchgeführt ist. Hierbei ist zu beachten, dass die zu sterilisierenden Gegenstände sauber und trocken sind und darüber hinaus in speziell gasdurchlässige Folien gepackt werden. Die chemische Sterilisation wird in der Regel bei thermolabilen Materialien, wie z.B. Endoskop-Optiken eingesetzt, so dass es erfindungsgemäß möglich ist, die Einführvorrichtung zusammen mit Endoskopen zu sterilisieren, welche zusammen mit der Einführvorrichtung verwendet werden sollen. Da hierbei chemische Stoffe verwendet werden müssen, die teuer in der Anschaffung und schwierig in der Handhabung und Entsorgung sind, stellt die Sterilisation mittels des Autoklaven das Mittel der Wahl dar.

Das sterilisierbare Material ist vorzugsweise ein nichtrostender Edelstahl oder ein Kunststoff, insbesondere PTFE (Polytetrafluorethylen) oder POM (Polyoxymethylen). Diese Materialien haben sich als besonders gut sterilisierbar erwiesen und können vielfach der Autoklavier-Routine unterzogen werden, ohne ihre Funktionsfähigkeit zu verlieren. Weiterhin können mit diesen Materialien sehr glatte Oberflächen erzeugt werden, so dass sich Krankheitserreger nicht oder nur in einem geringen Umfang an die Oberflächen anhaften können und daher leicht entfernbar sind.

Die Erfindung wird anhand eines bevorzugten Ausführungsbeispiels unter Bezugnahme auf die anhängenden Zeichnungen im Detail beschrieben. Es zeigen
- Figur 1: eine Prinzipskizze der erfindungsgemäßen Einführvorrichtung,
- Figur 2a: eine vergrößerte Darstellung eines rohrförmigen Körpers,
- Figur 2b: eine perspektivische Darstellung des in Figur 2a) dargestellten rohrförmigen Körpers,
- Figur 3: eine vergrößerte Darstellung des Schafts,
- Figur 4: eine vergrößerte Darstellung einer Feder,
- Figur 5: eine Prinzipskizze der erfindungsgemäßen Einführvorrichtung im gebrauchsfertigen Zustand gemäß einer ersten Ausführungsform, und
- Figur 6: eine Prinzipskizze der erfindungsgemäßen Einführvorrichtung im gebrauchsfertigen Zustand gemäß einer zweiten Ausführungsform.

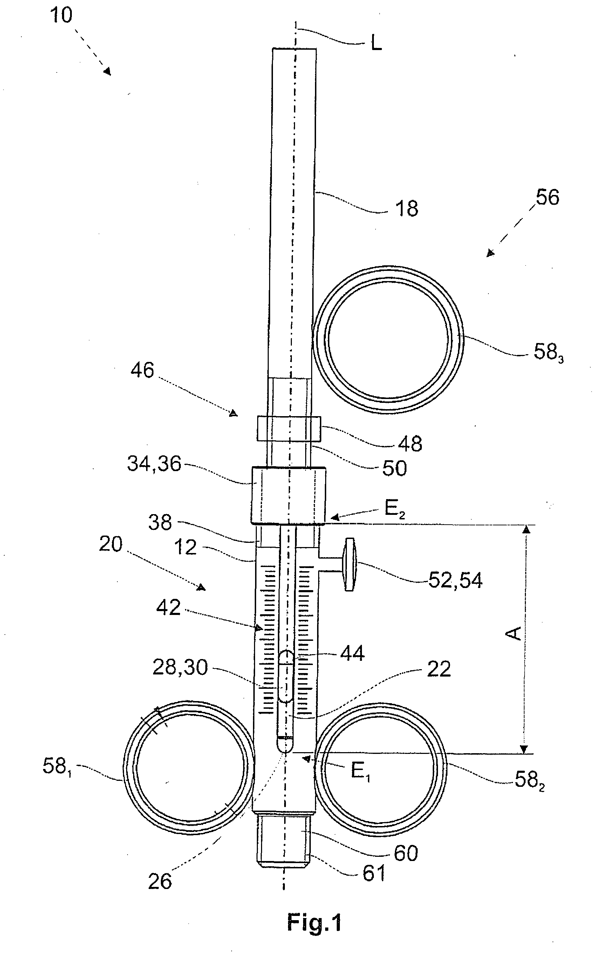

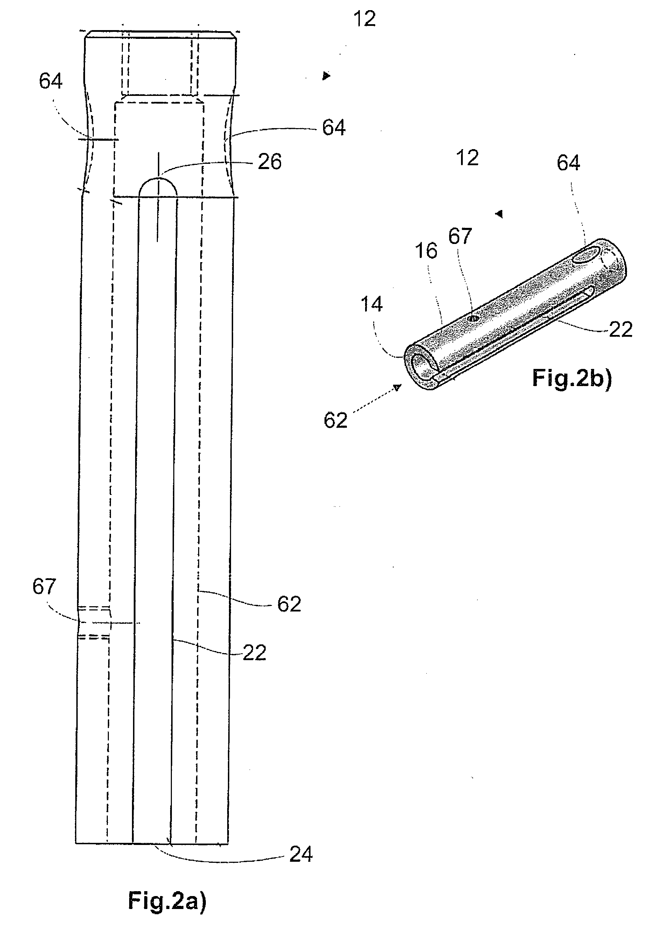

In Figur 1 ist eine erfindungsgemäße Einführvorrichtung 10 gemäß einem Ausführungsbeispiel dargestellt, die einen rohrförmigen Grundkörper 12 mit einer Längsachse L, einer Wandung 14 und einer Außenfläche 16 und einen im rohrförmigen Grundkörper 12 verschiebbar angeordneten Schaft 18 umfasst (vgl. Figur 2). Die Einführvorrichtung 10 umfasst weiterhin eine Einrichtung 20 zum Ermitteln der Position des Schafts 18 im rohrförmigen Grundkörper 12, die im dargestellten Beispiel als eine Ausnehmung 22 ausgeführt ist, welche die gesamte Wandung 14 des rohrförmigen Grundkörpers 12 durchdringt und durch welche der Schaft 18 und damit seine Position im rohrförmigen Grundkörper 12 sichtbar ist. Die Ausnehmung 22 weist ein offenes Ende 24 und ein geschlossenes Ende 26 auf (vgl. Figur 2).

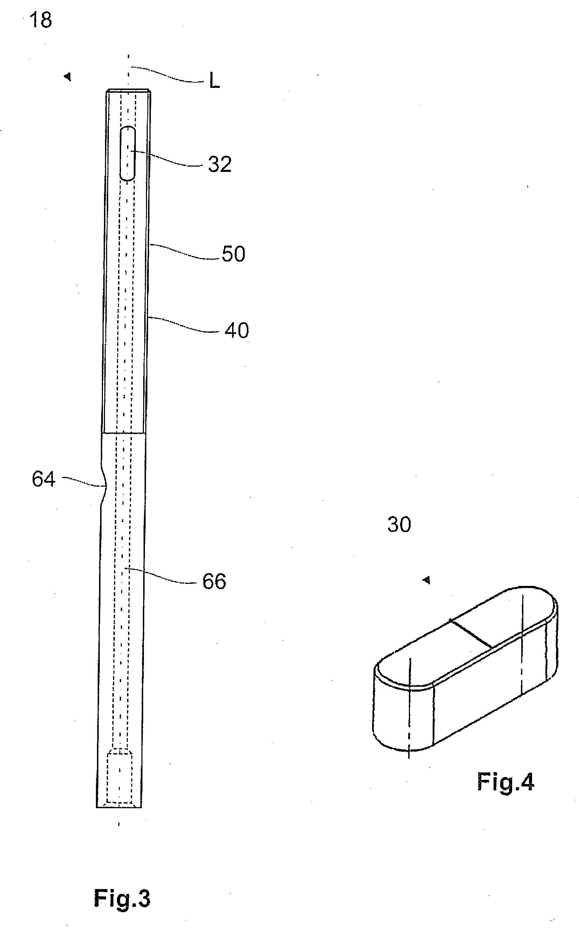

Der Schaft 18 umfasst einen Anschlag 28 zum Definieren einer ersten Endstellung E₁, der im dargestellten Beispiel als Stein oder Feder 30 (vgl. Figur 4) ausgeführt ist, die in eine Nut 32 des Schafts 18 einbringbar ist (vgl. Figur 3), um sie mit dem Schaft 18 lösbar zu verbinden. Die Feder 30 ist so gestaltet, dass sie in der Ausnehmung 22 entlang der Längsachse L bewegbar ist. Das offene Ende 24 ist mit einem Verschlusselement 34 verschließbar, welches im dargestellten Ausführungsbeispiel als Überwurfmutter 36 ausgeführt ist, die auf ein Gewinde 38 auf der Außenfläche 16 des rohrförmigen Körpers 12 aufschraubbar ist. Die Überwurfmutter 36 verhindert, dass der Schaft 18 unbeabsichtigt aus dem rohrförmigen Körper 12 herausgezogen wird. Sie legt weiterhin eine zweite Endstellung E₂ fest, die einen Abstand A von der ersten Endstellung E₁ aufweist. Der Schaft 18 kann zwischen der ersten und der zweiten Endstellung E₁, E₂ maximal um den Abstand A verschoben werden, solange die Überwurfmutter 36 aufgeschraubt ist, da die Feder 30 einerseits in der ersten Endstellung E₁ gegen das geschlossene Ende 24 der Ausnehmung 22 und andererseits in der zweiten Endstellung E₂ gegen die Überwurfmutter 36 anschlägt. Dieser Abstand beträgt vorzugsweise 35 mm.

Benachbart zur Ausnehmung 22 ist eine Skalierung 42 auf der Außenfläche 16 des rohrförmigen Körpers 12 vorgesehen, weiterhin weist die Feder 30 eine Markierung 44 auf, so dass die Position des Schafts 18 im rohrförmigen Körper 12 quantitativ ermittelt werden kann.

Ferner weist die erfindungsgemäße Einführvorrichtung 10 eine Einstelleinrichtung 46 auf, mit welcher die maximale Verschiebbarkeit des Schafts 18 im rohrförmigen Grundkörper 12 veränderbar ist. Die Einstelleinrichtung 46 dient dazu, den Abstand A zu verändern, sofern dies aufgrund von bestimmten Umständen, etwa bei anatomischen Besonderheiten der zu behandelnden Person, notwendig werden sollte. Hierzu weist die Einstelleinrichtung 46 eine Mutter 48 auf, welche auf ein weiteres Gewinde 50 auf der Außenfläche 40 des Schafts 18 aufschraubbar ist (vgl. Figur 3). Die Mutter 48 kann entlang des weiteren Gewindes 50 bewegt werden. Zum Verändern der ersten Endstellung E₁ wird die Mutter 48 an eine bestimmte Stelle auf dem weiteren Gewinde 50 bewegt. Wird der Schaft 18 nun im rohrförmigen Körper 12 ausgehend von der zweiten Endstellung E₂ in Richtung der ersten Endstellung E₁ bewegt, schlägt die Mutter 48 je nach Stellung auf dem weiteren Gewinde 50 gegen den rohrförmigen Körper 12 bzw. gegen die Überwurfmutter 36 bei einer mehr oder weniger weiten Verschiebung des Schafts 18 an. Es kann auch passieren, dass die Mutter 48 nicht gegen die Überwurfmutter 36 oder den rohrförmigen Körper 12 anschlägt, da sie sich in einer Position befindet, in der die Feder 30 zuerst gegen das geschlossene Ende 26 anschlägt. In jedem Fall wird der Abstand A nicht überschritten.

Ferner ist eine Fixiereinrichtung 52 vorgesehen, mit welcher die Position des Schafts 18 im rohrförmigen Körper 12 fixiert werden kann, um eine Positionsänderung zu verhindern. Im dargestellten Beispiel ist die Fixiereinrichtung 52 als Schraube 54 ausgeführt, welche senkrecht zur Längsachse L verläuft und die Wandung 14 des rohrförmigen Körpers 12 durchdringt. Ist der Schaft 18 in der gewünschten Position, wird die Schraube 54 angezogen, bis dass sie mit der Außenfläche 40 des Schafts 18 zur Anlage kommt und den Schaft 18 verspannt, so dass dieser in seiner Position fixiert ist.

Die erfindungsgemäße Einführvorrichtung 10 weist weiterhin eine Greifeinrichtung 56 auf, die im dargestellten Beispiel drei Ringe 58 umfasst, von denen zwei Ringe 58₁ und 58₂ am rohrförmigen Körper 12 und ein Ring 58₃ am Schaft 18 befestigt sind. Der Operateur kann die Einführvorrichtung 10 dadurch ergreifen, dass er beispielsweise den Zeige- und den Mittelfinger durch die Ringe 58₁, 58₂ und den Daumen durch den Ring 58₃ führt. Er kann so den Schaft 18 mit einer Hand beliebig zwischen der ersten und der zweiten Endstellung E₁ und E₂ hin-und her bewegen. Da üblicherweise der Zeige- und Mittelfinger einen geringeren Durchmesser als der Daumen aufweisen, kann der Ring 58₃ einen größeren Durchmesser haben.

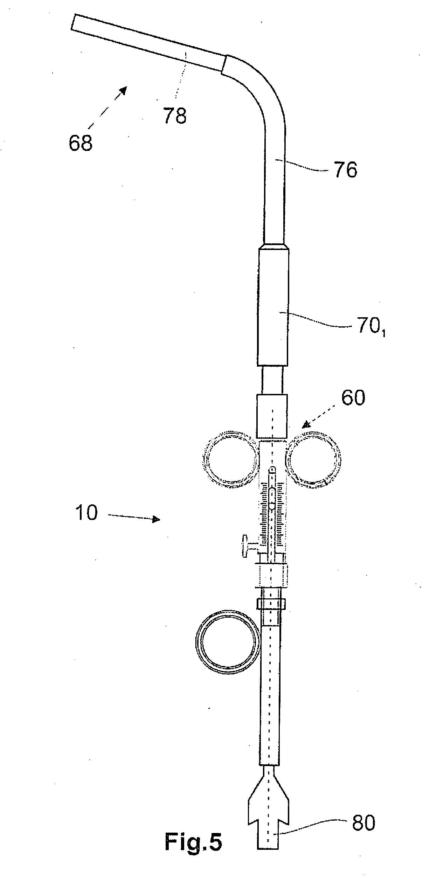

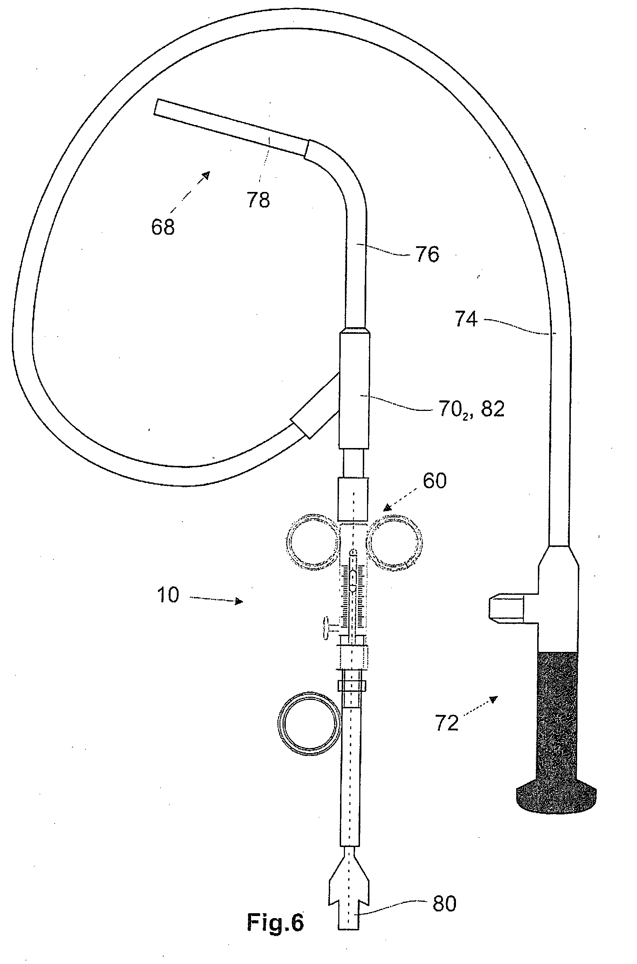

Weiterhin weist die Einführvorrichtung 10 eine Anschlusseinheit 60 auf, an welche eine weitere Einheit 70 angeschlossen werden kann (vgl. Figuren 5 und 6). Die Anschlusseinheit 60 kann ein Gewinde 61 umfassen oder als Luer Lock (nicht dargestellt) ausgeführt sein, um die weiteren Einheiten 70 lösbar mit der Einführvorrichtung 10 zu verbinden.

In Figur 2a) ist der rohrförmige Körper isoliert dargestellt. Der rohrförmige Körper weist eine Durchgangsbohrung 62 auf, in welcher der Schaft 18 bewegbar ist. Weiterhin ist die Ausnehmung 22 mit dem offenen und dem geschlossenen Ende 24, 26 erkennbar, welche die Wandung 14 durchdringt. Ferner ist eine Gewindebohrung 67 zu erkennen, die senkrecht zur Durchgangsbohrung 62 verläuft und in welche die Schraube 54 einschraubbar ist. Weiterhin sind Vertiefungen 64 vorgesehen, die zur Positionierung der Ringe 58 dienen, beispielsweise dann, wenn sie an den rohrförmigen Körper 12 angeschweißt werden sollen.

In Figur 2b) ist der in Figur 2a) dargestellte rohrförmige Körper 12 perspektivisch gezeigt.

In Figur 3 ist der Schaft 18 isoliert dargestellt, welcher eine weitere Durchgangsbohrung 66 aufweist, durch welche ein Ballonkatheter 68 (vgl. Figur 5) so durchgeführt wird, dass er vom Schaft 18 bewegt werden kann. Auch der Schaft 18 weist eine Vertiefung 64 auf, mit welcher der Ring 58 zu oben beschriebenen Zwecken positioniert werden kann. Ferner weist der Schaft 18 die Nut 32 auf, in welche die in Figur 4 dargestellte Feder 30 eingebracht werden kann.

In Figur 4 ist die Feder 30 isoliert dargestellt, welche in die Nut 32 des Schafts 18 einbringbar ist (vgl. Figur 3).

In Figur 5 ist die Einführvorrichtung 10 im gebrauchsfertigen Zustand dargestellt. Die Einführvorrichtung 10 ist mit Hilfe der Anschlusseinheit 60 mit der weiteren Einheit 70₁ gemäß einer ersten Ausführungsform verbunden. In dieser Ausführungsform ist die weitere Einheit 70₁ so ausgeführt, dass keine zusätzlichen Geräte wie ein Endoskop anschließbar sind (vgl. Figur 6). Die erste Ausführungsform stellt die einfachste Ausgestaltung der weiteren Einheit 70 dar und stellt eine Umgebung bereit, so dass problemlos weitere Geräte wie ein Endoskop zusammen mit der erfindungsgemäßen Einführvorrichtung 10 verwendet werden können, wozu nur die weitere Einheit 70₁ gemäß der ersten Ausführungsform durch eine weitere Einheit 70 gemäß einer weiteren Ausführungsform ausgetauscht werden muss (vgl. Figur 6). Das Austauschen kann auf einfache Weise mittels der Anschlussvorrichtung 60 geschehen, um die weitere Einheit 70 gemäß einer weiteren Ausführungsform mit der Einführvorrichtung 10 zu verbinden. In der ersten Ausführungsform kann die weitere Einheit 70₁ auch als Dummy oder als Handgriff bezeichnet werden, wodurch zum Ausdruck kommt, dass sie in der ersten Ausführungsform zwar nicht aktiv, beispielsweise als Endoskop, genutzt werden kann, in diesem Fall zum Anschließen weiterer Geräte, dennoch die Voraussetzungen schafft, dass dies mit nur wenig Aufwand ermöglicht wird.

Die weitere Einheit 70₁ hat eine Hülse 76 mit einer Krümmung, welche das Einführen in das zu behandelnde Gefäß oder die zu behandelnde Röhre erleichtert. Am freien Ende der Hülse 76 befindet sich ein Ballon 78 des Ballonkatheters 68, der mit dem Maß über das freie Ende übersteht, das mit der Einführvorrichtung 10 vorgegeben wird. Der Ballon 78 ist mit einer nicht dargestellten Einheit aufblasbar, die an einem äußeren Ende 80 des Ballonkatheters 68 anschließbar ist.

Um die mit dem Ballonkatheter 68 vorgenommene Behandlung und insbesondere das Einführen der Hülse 76 und des Ballons 78 über den Nasenrachen in die Eustachische Röhre zu verfolgen, kann eine weitere, unabhängig vom Ballonkatheter betätigbare Beobachtungseinheit zum Beispiel mit einer starren Optik verwendet werden, die ebenfalls in den Nasenrachen eingeführt wird. Hierdurch wird es ermöglicht, den Einführvorgang von einem geeigneten, fixen Ort aus in verschiedene Blickrichtungen zu beobachten.

Auch in Figur 6 ist die Einführvorrichtung 10 im gebrauchsfertigen Zustand dargestellt, allerdings ist sie mit der weiteren Einheit 70₂ gemäß einer zweiten Ausführungsform verbunden. Die weitere Einheit 70₂ gemäß der zweiten Ausführungsform ist als eine Zusammenführeinrichtung 82 ausgeführt, so dass zusätzliche Geräte anschließbar sind. Die Hülse 76 sowie der Ballon 78 entsprechen denjenigen, die in Figur 5 dargestellt sind.

An die weitere Einheit 70₂ kann beispielsweise ein Endoskop 72 angeschlossen werden, so dass die mit dem Ballonkatheter 68 vorgenommene Behandlung in Echtzeit beobachtet werden kann. Das Endoskop 72 weist einen flexiblen Schlauch 74 auf, der einen Licht- und einen Beobachtungskanal umfasst, der in die weitere Einheit 70₁ mündet.

Im Gegensatz zur Verwendung einer zusätzlichen Beobachtungseinheit, die nicht mit der erfindungsgemäßen Einführvorrichtung zusammenwirkt, ist der Geräteaufwand etwas geringer. Allerdings bieten die Endoskope, die über die weitere Einheit 70₂ anschließbar sind, im Allgemeinen eine im Gegensatz zu zusätzlichen Beobachtungseinheiten mit einer rigiden oder starren Optik geringe Auflösung, so dass Details des Umfelds, in dem sich der Ballon 78 befindet, nicht erkennbar sind. Ferner ist die Orientierung im Umfeld schwieriger, da keine fixen Bezugspunkte ermittelt werden können, so dass im Allgemeinen die Kontrolle der Behandlung mit einer weiteren Beobachtungseinheit besser ist. Je nach Einzelfall kann die eine oder die andere Ausführungsform vorteilhafter sein. Die erfindungsgemäße Einführvorrichtung kann flexibel an den jeweiligen Einzelfall angepasst werden und ist problemlos erweiterbar.

### Bezugszeichenliste

- 10: Einführvorrichtung
- 12: ringförmiger Grundkörper
- 14: Wandung
- 16: Außenfläche von 12
- 18: Schaft

- 20: Einrichtung
- 22: Ausnehmung
- 24: offenes Ende
- 26: geschlossenes Ende
- 28: Anschlag

- 30: Feder
- 32: Nut
- 34: Verschlusselement
- 36: Überwurfmutter
- 38: Gewinde

- 40: Außenfläche von 18
- 42: Skalierung
- 44: Markierung
- 46: Einstellvorrichtung
- 48: Mutter

- 50: weiteres Gewinde
- 52: Fixiereinrichtung
- 54: Schraube
- 56: Greifeinrichtung
- 58: Ring

- 60: Anschlusseinrichtung
- 62: Durchgangsbohrung
- 64: Vertiefung
- 66: weitere Durchgangsbohrung
- 67: Gewindebohrung
- 68: Ballonkatheter

- 70₁, 70₂: weitere Einheit
- 72: Endoskop
- 74: Schlauch
- 76: Hülse
- 78: Ballon

- 80: äußeres Ende
- 82: Zusammenführeinrichtung

- L: Längsachse
- E₁: erste Endstellung
- E₂: zweite Endstellung
- A: Abstand

## Patentansprüche

1. Einführvorrichtung für einen Ballonkatheter (68), umfassend
- einen rohrförmigen Grundkörper (12),
- einen in den rohrförmigen Grundkörper (12) einbringbaren und im rohrförmigen Grundkörper (12) verschiebbaren Schaft (18) zum Bewegen des Ballonkatheters (68),
- eine Einrichtung (20) zum Ermitteln der Position des Schafts (18) im rohrförmigen Grundkörper (12), wobei
- der rohrförmige Grundkörper (12) eine Wandung (14) aufweist und die Einrichtung (20) eine die Wandung (14) des rohrförmigen Grundkörpers (12) vollständig durchdringende Ausnehmung (22) zum Ermitteln der Position des Schafts (18) und
- einen mit der Ausnehmung (22) zusammenwirkenden Anschlag (28) zum Definieren einer ersten Endstellung (E1) des Schafts (18) im rohrförmigen Grundkörper (12) umfasst,
**dadurch gekennzeichnet, dass** die Ausnehmung (22) ein offenes Ende (24) und ein geschlossenes Ende (26) aufweist, wobei der Schaft (18) mit dem Anschlag (28) durch das offene Ende (24) in den rohrförmigen Grundkörper (12) einbringbar ist.

2. Einführvorrichtung nach Anspruch 1,
**dadurch gekennzeichnet, dass** der Anschlag (28) als eine Feder (30) ausgeführt ist, die in eine korrespondierende Nut (32) des Schafts (18) einbringbar ist.

3. Einführvorrichtung nach Anspruch 2,
**gekennzeichnet durch** ein Verschlusselement (34), mit dem das offene Ende (24) wahlweise verschließbar ist.

4. Einführvorrichtung nach Anspruch 3,
**dadurch gekennzeichnet, dass** das Verschlusselement (34) als eine Überwurfmutter (36) ausgebildet ist, die auf ein Gewinde (38) auf einer Außenfläche (40) des rohrförmigen Grundkörpers (12) aufschraubbar ist.

5. Einführvorrichtung nach Anspruch 2, 3 oder 4,
**dadurch gekennzeichnet, dass** die Einrichtung (12) eine Skalierung (42) zum Ermitteln der Position des Schafts (18) im rohrförmigen Grundkörper (12) und die Feder (30) eine mit der Skalierung (42) zusammenwirkende Markierung (44) zum Ermitteln der Position des Schafts (18) im rohrförmigen Grundkörper (12) aufweisen.

6. Einführvorrichtung nach einem der vorherigen Ansprüche, **gekennzeichnet durch** eine Einstelleinrichtung (46), mit welcher die maximale Verschiebbarkeit des Schafts (18) im rohrförmigen Grundkörper (12) einstellbar ist.

7. Einführvorrichtung nach einem der vorherigen Ansprüche, **gekennzeichnet durch** eine Fixiereinrichtung (52) zum Fixieren des Schafts (18) im rohrförmigen Grundkörper (12).

8. Einführvorrichtung nach einem der vorherigen Ansprüche, **gekennzeichnet durch** eine Greifeinrichtung (56) zum Verschieben des Schafts (18) im rohrförmigen Grundkörper (12).

9. Einführvorrichtung nach Anspruch 8,
**dadurch gekennzeichnet, dass** die Greifeinrichtung (56) eine Anzahl von Ringen (58) aufweist, von der mindestens ein Ring (58) am rohrförmigen Grundkörper (12) und mindestens ein Ring (58) am Schaft (18) befestigt sind.

10. Einführvorrichtung nach einem der vorherigen Ansprüche, **gekennzeichnet durch** eine Anschlusseinrichtung (60) zum lösbaren Anschließen einer weiteren Einheit (70) an die Einführvorrichtung (10).

11. Einführvorrichtung nach Anspruch 10,
**dadurch gekennzeichnet, dass** die weitere Einheit (70) als Zusammenführeinrichtung (82) ausgeführt ist.

12. Einführvorrichtung nach einem der vorherigen Ansprüche, **dadurch gekennzeichnet, dass** die Einführvorrichtung (10) aus sterilisierbarem Material wie ein nichtrostender Edelstahl oder ein Kunststoff, insbesondere PTFE oder POM besteht.

## Claims

1. Intubation device for a balloon catheter (68), comprising
- a tubular base body (12),
- a shaft (18) for moving the balloon catheter (68), the shaft being insertable into the tubular base body (12) and movable inside the tubular base body (12),
- a setup (20) for determining the position of the shaft (18) in the tubular base body (12), wherein
- the tubular base body (12) comprises a wall (14) and the setup (20) comprises
- a recess (22) that completely runs through the wall (14) of the tubular base body (12) for determining the position of the shaft (18),
- and a backstop (28) that is interacting with the recess (22) for defining a first
end position (E1) of the shaft (18) in the tubular base body (12), **characterized in that** the recess (22) comprises an open end (24) and a closed end (26), wherein the shaft (18) together with the backstop (28) are insertable into the tubular base body (12) via the open end (24).

2. Intubation device according to claim 1,
**characterized in that** the backstop (28) is embodied as a tongue (30) that is insertable into a corresponding groove (32) of the shaft (18).

3. Intubation device according to claim 2,
**characterized by** a locking element (34) for selectively closing the open end (24).

4. Intubation device according to claim 3,
**characterized in that** the locking element (34) is embodied as a cap nut (36) that is screwable onto a thread (38) provided on the outer surface (40) of the tubular base body (12).

5. Intubation device according to one of the claims 2, 3 or 4,
**characterized in that** the device (12) comprises a scale (42) for determining the position of the shaft (18) in the tubular base body (12) and the tongue (30) comprises a marker (44) cooperating with the scale (42) for determining the position of the shaft (18) in the tubular base body (12).

6. Intubation device according to one of the preceding claims,
**characterized by** an adjustment device (46) for adjusting the maximum clearance of the shaft (18) inside the tubular base body (12).

7. Intubation device according to one of the preceding claims,
**characterized by** a fixing device for fixing the shaft (18) in the tubular base body (12).

8. Intubation device according to one of the preceding claims,
**characterized by** a grabbing device (56) for moving the shaft (18) inside the tubular base body (12).

9. Intubation device according to claim 8,
**characterized in that** the grabbing device (56) comprises a number of rings (58) of which at least one ring (58) is fastened on the tubular base body (12) and at least one ring (58) is fastened on the shaft (18).

10. Intubation device according to one of the preceding claims,
**characterized by** a connecting unit (60) for releasably connecting a further unit (70) with the intubation device (10).

11. Intubation device according to claim 10,
**characterized in that** the further unit (70) is embodied as a merging device (82).

12. Intubation device according to one of the preceding claims,
**characterized in that** the intubation device (10) consists of a sterilizable material such as stainless steel or plastic, in particular PTFE or POM.

## Revendications

1. Dispositif d'introduction pour un cathéter à ballonnet (68), comprenant
- un corps de base tubulaire (12),
- une tige (18) pouvant être introduite dans le corps de base tubulaire (12) et pouvant coulisser dans le corps de base tubulaire (12) pour déplacer le cathéter à ballonnet (68),
- un moyen (20) pour déterminer la position de la tige (18) dans le corps de base tubulaire (12),
- le corps de base tubulaire (12) présentant une paroi (14) et le moyen (20) comprenant un évidement (22) traversant complètement la paroi (14) du corps de base tubulaire (12) pour déterminer la position de la tige (18) et
- une butée (28) coopérant avec l'évidement (22) pour défmir une première
position extrême (E1) de la tige (18) dans le corps de base tubulaire (12), **caractérisé en ce que** l'évidement (22) présente une extrémité ouverte (24) et une extrémité fermée (26), la tige (18) pouvant être introduite avec la butée (28) dans le corps de base tubulaire (12) à travers l'extrémité ouverte (24).

2. Dispositif d'introduction selon la revendication 1,
**caractérisé en ce que** la butée (28) est réalisée sous la forme d'un ressort (30) qui peut être introduit dans une rainure (32) correspondante de la tige (18).

3. Dispositif d'introduction la revendication 2,
**caractérisé par** un élément de fermeture (34) avec lequel l'extrémité ouverte (24) peut être fermée au choix.

4. Dispositif d'introduction selon la revendication 3,
**caractérisé en ce que** l'élément de fermeture (34) est réalisé sous la forme d'un écrou-raccord (36) qui peut être vissé sur un filetage (38) sur une surface extérieure (40) du corps de base tubulaire (12).

5. Dispositif d'introduction selon la revendication 2, 3 ou 4,
**caractérisé en ce que** le moyen (12) présente une échelle graduée (42) pour déterminer la position de la tige (18) dans le corps de base tubulaire (12) et le ressort (30) présente un repère (44) qui coopère avec l'échelle graduée (42) pour déterminer la position de la tige (18) dans le corps de base tubulaire (12).

6. Dispositif d'introduction selon une des revendications précédentes, **caractérisé par** un moyen de réglage (46) avec lequel la capacité maximale de coulissement de la tige (18) dans le corps de base tubulaire (12) peut être réglée.

7. Dispositif d'introduction selon une des revendications précédentes, **caractérisé par** un moyen de fixation (52) pour fixer la tige (18) dans le corps de base tubulaire (12).

8. Dispositif d'introduction selon une des revendications précédentes, **caractérisé par** un moyen de préhension (56) pour faire coulisser la tige (18) dans le corps de base tubulaire (12).

9. Dispositif d'introduction selon la revendication 8,
**caractérisé en ce que** le moyen de préhension (56) présente une pluralité de bagues (58) dont au moins une bague (58) est fixée au corps de base tubulaire (12) et au moins une bague (58) à la tige (18).

10. Dispositif d'introduction selon une des revendications précédentes, **caractérisé par** un moyen de raccordement (60) pour le raccordement détachable d'une autre unité (70) au dispositif d'introduction (10).

11. Dispositif d'introduction selon la revendication 10,
**caractérisé en ce que** l'autre unité (70) est réalisée sous la forme d'un moyen de combinaison (82).

12. Dispositif d'introduction selon une des revendications précédentes, **caractérisé en ce que** le dispositif d'introduction (10) est réalisé dans un matériau stérilisable comme un acier inoxydable ou une matière plastique, en particulier PTFE ou POM.
